(19) Europäisches Patentamt
European Patent Office
Office européen des brevets

(11) **EP 2 954 867 A1**

(12) **EUROPÄISCHE PATENTANMELDUNG**

(43) Veröffentlichungstag:
16.12.2015 Patentblatt 2015/51

(51) Int Cl.:
*A61B 18/14* (2006.01)    *A61N 1/06* (2006.01)
*A61N 1/05* (2006.01)    *A61B 5/00* (2006.01)
*G01L 1/24* (2006.01)    *A61B 19/00* (2006.01)

(21) Anmeldenummer: 15168296.0

(22) Anmeldetag: **20.05.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(30) Priorität: **11.06.2014 US 201462010466 P**

(71) Anmelder: VascoMed GmbH
79589 Binzen (DE)

(72) Erfinder: **Bitzer, Andreas**
**8032 Zürich (CH)**

(74) Vertreter: **Galander, Marcus et al**
**Biotronik SE & Co. KG**
**Corporate Intellectual Properties**
**Woermannkehre 1**
**12359 Berlin (DE)**

(54) **PLANAR AUFGEBAUTES LOGICBOARD FÜR ABLATIONSKATHETER MIT KRAFTMESSFUNKTIONALITÄT**

(57) Sensor-Anschlusselement eines Katheters oder einer Elektrodenleitung, welches ein plattenartiges Substrat mit mindestens einer Ausnehmung und schichtartigen elektrischen Leitern mit Sensor-Anschlussflächen und/oder Aktor-Anschlussflächen auf oder unterhalb mindestens einer Sensoroberfläche aufweist.

FIG. 1A

**Beschreibung**

Die Erfindung betrifft ein Sensor-Anschlusselement eines Katheters oder einer Elektrodenleitung sowie einen Katheter und eine Elektrodenleitung, der/die eines solches Sensor-Anschlusselement enthält. Der Begriff "Sensor-Anschlusselement" ist nachfolgend einerseits als Anschlusselement eines Sensors in einem Katheter oder einer Elektrodenleitung, darüber hinaus aber auch im Sinne von Sensor-/Anschlusselement, also in einem breiteren Sinne zunächst als Sensorelement, aber auch als ein von Sensorfunktionen unabhängiges Anschlusselement sonstiger Funktionseinheiten eines Katheters oder einer Elektrodenleitung, insbesondere von Stimulations- oder Ablationselektroden oder sonstigen Aktoren, zu verstehen.

Die Erfindung hat ihren Ausgangspunkt im medizintechnischen Spezialgebiet der sogenannten Ablationskatheter, mit denen Bereiche biologischem Gewebes verödet bzw. Gewebeteile abgetragen werden, und noch spezieller bei Ablationskathetern mit Kontaktkraftmessung.

Es ist ein Ablationskatheter ("TactiCath", Fa. Endosense) bekannt, welches die Messung einer auf das distale Katheterende einwirkenden Kraft - im Anwendungsfall also der erwähnten Kontaktkraft - nach Betrag und Richtung während eines Ablationsvorganges ermöglicht. Dieser Katheter nutzt das Prinzip des sogenannten FBG (Faser-Bragg-Gitter)-Sensors, wobei drei Fasern mit jeweils einem FBG-Sensor am Faserende die für eine 3D-Kraftmessung benötigte Gruppe von Sensoren bilden, die zur gemeinsamen Messsignalverarbeitung an eine Signalverarbeitungseinheit einschließbar sind. Die Sensoren sind in einem Winkelabstand von 120° außen auf einem verformbaren Zylinder aufgebracht.

In der US 2008/0285909 A1 wird die Funktionsweise von FBG-Sensoren zur Bestimmung von Verdrillungen oder Krümmungen des Katheterkörpers ausführlich beschrieben, und auch die Funktionsweise des vorgenannten Kraftsensors mit mehreren FBG-Fasern auf einem verformbaren Zylinder ist in dieser Druckschrift erläutert.

In der auf die Anmelderin zurückgehenden EP 2 491 883 A1 wird ein in seinem Aufbau vereinfachter Ablationskatheter mit Kontaktkraftmessung beschrieben, der eine einzelne FBG-Faser mit einer Mehrzahl von in Längsrichtung beabstandet voneinander angeordneten Sensorbereichen enthält. Diese Sensorbereiche sind derart angeordnet und ausgeführt und werden in geeigneter Weise ausgelesen, dass die räumlichen Kraftkomponenten einer auf das Katheterende einwirkenden Kraft erfasst werden können und auch die Gewinnung von Temperatursignalen zur Korrektur der Kraftmesssignale möglich ist.

Unabhängig von diesem Messprinzip sind auch andere Lösungen für eine Kontaktkraftmessung an einem Führungsdraht oder Katheter bekannt, bspw. unter Einsatz eines optischen Sensors, wie in der WO 2009/007857 A2 beschrieben, oder unter Einsatz eines Halbleitersensors an der Spitze eines Führungsdrahtes, wie in der WO 2008/503307 A2 beschrieben.

Außerhalb des genannten technischen Gebietes sind mehrachsige Kraft- und Drehmomentsensoren in verschiedensten Ausführungen seit langem bekannt, und es gibt auch Realisierungsformen, bei denen gedruckte Leiterplatten Einsatz finden; vgl. WO 2010/088922 A1.

Bei der Konstruktion serientauglicher Sensor-Anschlusselemente der genannten Art wurde die Erkenntnis gewonnen, dass die bisher bekannten Aufbauten aus technologischer Sicht nicht optimal sind und insbesondere der Automatisierung der Herstellung gewisse Grenzen setzen, die zu relativ hohen Produktionskosten führen.

Der Erfindung liegt daher die Aufgabe zugrunde, ein insbesondere hinsichtlich seiner Eignung für die Serienproduktion und somit hinsichtlich der Produktionskosten verbessertes Sensor-Anschlusselement und somit letztlich auch einen kostengünstigeren Katheter oder eine Elektrodenleitung mit einem solchen Sensor-Anschlusselement bereitzustellen.

Diese Aufgabe wird grundsätzlich durch ein Sensor-Anschlusselement mit den Merkmalen des Anspruchs 1 und hinsichtlich der hieraus abgeleiteten medizintechnischen Produkte durch einen Katheter mit den Merkmalen des Anspruchs 12 oder eine Elektrodenleitung mit den Merkmalen des Anspruchs 13 gelöst. Zweckmäßige Fortbildungen des Erfindungsgedankens sind Gegenstand der jeweiligen abhängigen Ansprüche.

Die Erfindung schließt neben der oben erwähnten Erkenntnis den Gedanken ein, von einem zumindest im Herstellungsprozess planaren, plattenartigen Substrat und schichtartigen elektrischen Leitern mit Sensor-Anschlussflächen und/oder Aktor-Anschlussflächen auf oder unterhalb mindestens einer Sensoroberfläche auszugehen.

Dies ermöglicht, verglichen mit bekannten Konstruktionen, eine geringere Komplexität des Herstellungsprozesses und eine weitgehend automatisierte Herstellung, unter Einsatz bekannter Techniken der Fotolithografie sowie marktgängiger Bestückungsmaschinen. Die Prozesse lassen sich weitgehend parallelisieren.

Des Weiteren ist, im Hinblick auf eine zuverlässige Funktion und Langzeitstabilität des fertigen Produkts, insbesondere von Kraftumformerelementen mit erfindungsgemäßem Aufbau, ein wesentlicher Vorteil in geringeren Anforderungen an die Fertigungstoleranz zu sehen. Mit dem erfindungsgemäßen Aufbau werden nämlich aufeinander gleitende Flächen, insbesondere Zylinderoberflächen, grundsätzlich vermieden, wodurch Anforderungen hinsichtlich der Herstellungstoleranz der zueinander passenden Zylindergrößen entfallen. Da typischerweise jeder erfindungsgemäß hergestellte Sensor (insbesondere Kraftumformer) individuell kalibriert wird, sind insgesamt Fertigungstoleranzen weniger entscheidend als bei bekannten Produkten.

Bisherige Kraftmesskatheter und ähnliche Produkte haben das Problem, dass auf engstem Raum sehr viele elektrische Leitungen und Schlauche verlegt sind, welche sich bezüglich Deformation nicht linear verhalten. Da das Messverfahren mit Dehnungsmessung auf Deformation beruht wirken sich diese Effekte störend aus. Bei dem hier beschriebenen erfindungsgemäßen Aufbau sind Leitungen extrem miniaturisiert, sodass diese Störungen deutlich geringer ausfallen.

Der Aufbau derzeit gebräuchlicher Katheter erfolgt von Hand, und der Katheter wird aus mehreren Bauteilen zusammengesetzt. Da die Produktion von Hand eine relativ hohe Toleranz verursacht, gegenüber der Genauigkeit welche mit Maschinen erreicht werden kann, ist der Ausschuss relativ groß. Die hier beschriebene Erfindung ermöglicht es, dass der Handmontage-Prozess durch ein Spritzgussverfahren ersetzt werden kann.

Gebräuchliche Elektrodenleitungen für Herzschrittmacher und automatische Defibrillatoren sind mit feinen metallischen Ringen versehen zur Ableitung von Herzsignalen. Diese müssen von Hand aufgefädelt und verdrahtet werden. Die hier vorgestellte Erfindung ermöglicht es in einer speziellen Ausführung, dass die Elektroden in technologisch einfacher Weise als planare Leiterfahnen vorgebildet und vollautomatisch verkabelt positioniert und am Leitungskörper angebracht werden können.

In einer ersten Ausgestaltung der Erfindung ist das plattenartige Substrat im Gebrauchszustand eben. Alternativ kann das plattenartige Substrat im Gebrauchszustand insbesondere in die Form eines Hohlzylinders oder Hohlzylinderabschnitts, gebogen sein. Bei der Herstellung eines Katheters oder einer Elektrodenleitung, die typischerweise eine zylindrische Außenform haben, kann das Substrat also zum einen radial angeordnet sein und zum anderen an der Innenwand des Leitungskörpers anliegen.

In einer weiteren Ausführung der Erfindung ist das Substrat mehrschichtig aufgebaut, und es hat zwei oder mehr Ebenen, in denen schichtartige elektrische Leiter angeordnet sind. Durch das Vorsehen mehrerer Leitungsebenen lässt sich, wie aus der Technik elektrischer Leiterplatten an sich bekannt, eine Mehrzahl verschiedener Signale störungsfrei übertragen.

In einer weiteren Ausführung der Erfindung ist das Substrat aus einem drucksteifen Material gebildet, und es weist mindestens je eine Ausnehmung in beiden Seitenkanten und in mindestens einer Oberfläche auf, die dem Sensor-Anschlusselement eine vorbestimmte Biegeelastizität verleihen. Hier handelt es sich um eine Ausführung, die speziell zur Bildung eines Kraftmessfühlers und damit letztlich zur Herstellung eines Kraftmesskatheters geeignet ist. Insbesondere sind zur Realisierung der Funktionalität gegeneinander in Längsrichtung des Substrats versetzte und sich bis über die Mittellinie erstreckende Ausnehmungen in den Seitenkanten vorgesehen, derart, dass das Sensor-Anschlusselement eine vorbestimmte Druck-Elastizität gegenüber in Längsrichtung wirkenden Kräften aufweist.

In einer weiteren Ausgestaltung ist vorgesehen, dass die Ausnehmung oder mindestens eine von mehreren Ausnehmungen rechteckigen oder trapezförmigen Querschnitt hat.

Im Hinblick auf verschiedene Realisierungsformen und Funktionen der erfindungsgemäß aufgebauten Produkte sinnvolle materialseitige Ausführungen können solche sein, bei denen das Substrat ein Metall oder Halbleitermaterial, insbesondere Silizium, und/oder eine Keramik, insbesondere Aluminiumoxid-Keramik, und/oder ein Kunststoffmaterial, insbesondere Epoxidharz, aufweist. Die Wahl des konkreten Materials kann von den technologischen Möglichkeiten eines Herstellers ebenso abhängen wie von den Gesamtabmessungen des Produkts oder von Kostengrenzen.

Eine aus derzeitiger Sicht sehr nutzbringende Ausführung ist eine solche, bei der auf dem Substrat in zu den Seitenkanten paralleler Anordnung ein FBG-Sensor angebracht ist und derart ausgelesen wird, dass das Sensor-Anschlusselement einen 3D-Kraftsensor bildet. Eine alternative Realisierung eines Kraftmesssensors sieht vor, dass die schichtartigen Leiter auf dem Substrat derart ausgebildet und wahlweise mit zusätzlichen Funktionselementen versehen sind, dass das Sensor-Anschlusselement einen elektrischen Dehnungsmessstreifen bildet.

Speziell im Hinblick auf die technologisch einfache und kostengünstige Fertigung von Elektrodenleitungen ist eine Ausführung von besonderem Interesse, bei der auf mindestens einer Aktor-Anschlussfläche eine Leiterfahne zur Realisierung einer Abfühl- und/oder Stimulationselektrode einer Elektrodenleitung aufgebracht ist. Bei einer Ablationselektrodenleitung kann nahe einer kurzen Seitenfläche des Substrats, die in Gebrauchslage am distalen Ende des Katheters zu liegen kommt, eine Anschlussfläche für eine Ablationselektrode aufgebracht sein.

In einer Ausgestaltung der mit dem erfindungsgemäßen Sensor-Anschlusselement aufgebauten Elektrodenleitung ist der Leitungskörper in einem distalen Abschnitt, in dem das Sensor-Anschlusselement angeordnet, druck- und biegeweich ausgeführt, derart, dass er das Ansprechen des 3D-Kraftsensors oder Dehnungsmessstreifens auf externe Kräfte nicht verfälscht.

In einer Ausgestaltung dieser Elektrodenleitung, die auf einer weiter oben beschriebenen Ausgestaltung des Sensor-Anschlusselementes aufbaut, hat die Leitung mindestens eine Ringelektrode, die durch Umwickeln des Leitungskörpers mit einer auf eine Aktor-Anschlussfläche des Sensor-Anschlusselementes aufgebrachte Leiterfahne gebildet ist.

Vorteile und Zweckmäßigkeiten der Erfindung ergeben sich im Übrigen aus der nachfolgenden Beschreibung von Ausführungsbeispielen anhand der Figuren. Von diesen zeigen:

Fig. 1a und 1b    perspektivische Ansichten eine schematische Gesamtansicht eines Sensor-Anschlusselementes bzw. eines distalen Endabschnittes eines entsprechenden Ablationskatheters gemäß der Erfindung in der Ausführung als Kraftumformerelement, beispielsweise in einer Ablationselektrodenleitung,

Fig. 2a bis 2c    Detailansichten des Sensor-Anschlusselementes gemäß Fig. 1 und

Fig. 3a bis 3c    skizzenartige Detailansichten des Substrats des Sensor-Anschlusselementes (Kraftumformers) im Ruhezustand bzw. unter verschiedenen Krafteinwirkungen, zur Erläuterung der Funktion und Kalibrierung.

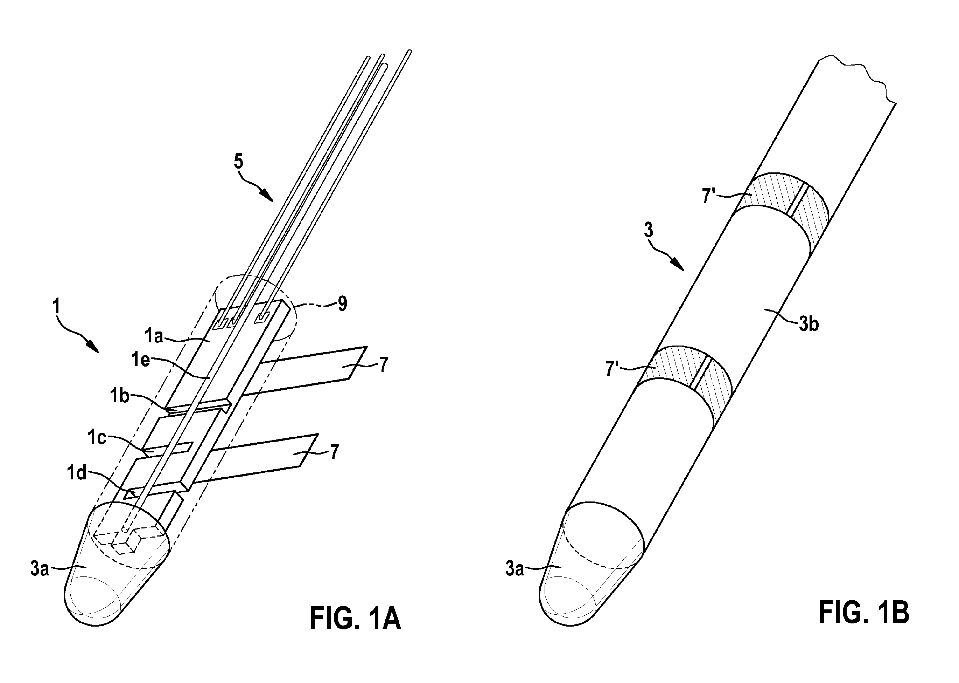

Fig. 1a zeigt ein planar aufgebautes Kraftumformerelement 1, montiert an einer Katheterspitze 3a einer (in dieser Figur nicht zu erkennenden) Ablationselektrodenleitung, und versehen mit aufgelöteten oder angebondeten Zuleitungen 5 sowie zwei rückseitig aufgepressten Leiterfahnen 7. Gestrichelt dargestellt ist eine im Gebrauchszustand vorhandene weich-elastische Kunststoffumspritzung 9. Das Kraftumformerelement 1 umfasst im Wesentlichen ein plattenförmiges Substrat 1a mit drei, im Querschnitt jeweils rechteckigen Ausnehmungen (etwa Ausfräsungen oder Laser-Gräben) 1b, 1c und 1d und einen mittig in Längserstreckung des Substrats fest verlegten FBG-Sensor 1e. Weitere Erläuterungen zum Aufbau und zur Funktionsweise des Kraftumformers finden sich weiter unten.

Fig. 1b zeigt eine Außenansicht des distalen Endabschnittes einer Ablationselektrodenleitung 3 mit der bereits erwähnten Katheterspitze 3a, die mit dem Kraftumformer in Wirkverbindung steht, und einem sich daran anschließenden weich-elastischen Leitungskörper 3b, beispielsweise aus einem für Katheter oder Elektrodenleitungen gebräuchlichen Silikonmaterial. Auf dem Leitungskörper 3b sind in Abstand von der Katheterspitze bzw. dem distalen Ende und voneinander beabstandet zwei Ringelektroden 7' angeordnet, die durch Umwickeln des Leitungskörpers mit den in Fig. 1a gezeigten Leiterfahnen 7 gebildet sind.

Im Übrigen sind Ablationselektrodenleitungen bzw. Ablationskatheter an sich bekannt, so dass von einer genaueren Beschreibung hier abgesehen werden kann. Es sei jedoch darauf hingewiesen, dass für die Ausführung mit einem integrierten Kraftumformer gemäß Fig. 1a auf eine ausreichend weich-elastische Umhüllung zu achten ist, um die Abfühlcharakteristik des Kraftumformers bzw. Dehnmessstreifens nicht nachteilig zu beeinflussen.

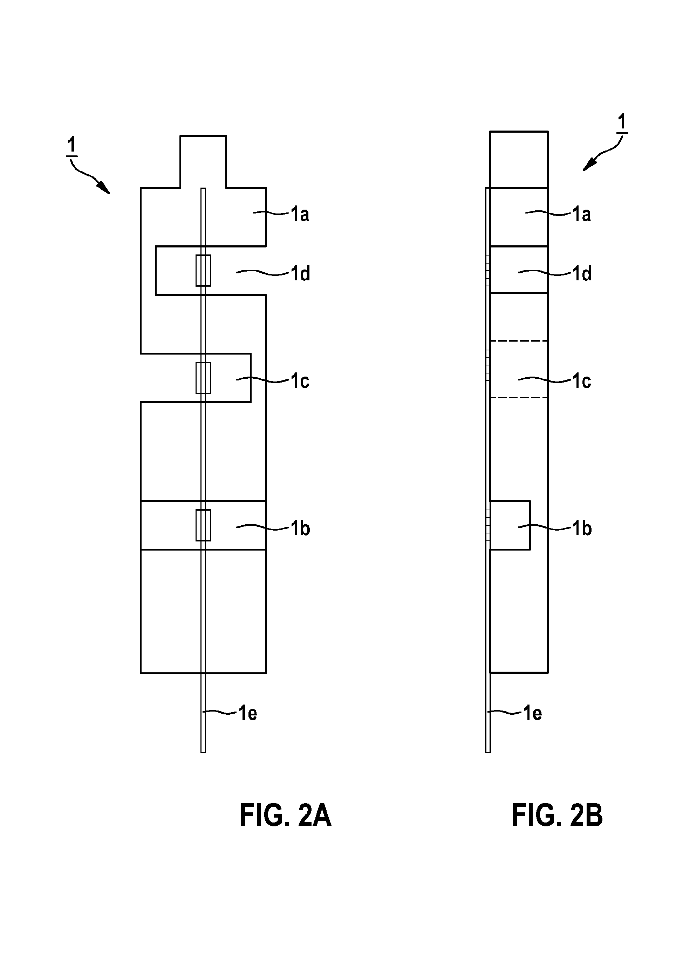

Fig. 2a und 2b zeigen den Kraftumformer 1, der aus dem Substrat 1a und dem FBG-Sensor 1e besteht, etwas genauer. Es ist zu erkennen, dass die drei als Sensorbereiche wirkenden (nicht separat mit Bezugsziffern bezeichneten) Sensorbereiche der FBG-Faser 1e jeweils einer der quaderförmigen bzw. im Querschnitt rechteckigen Ausnehmungen 1b, 1c, 1d des Substrats 1a positionsmäßig zugeordnet sind. Aufbau und Wirkungsweise eines solchen Einzelfaser-FBG-Sensors (nachfolgend auch bezeichnet als FBG-Faser) sind aus der EP 2 491 883 A1 bekannt und bedürfen hier keiner genaueren Beschreibung. Es ergeben sich jedoch in Verbindung mit dem planaren Substrat 1a gegenüber bisher bekannten zylindrischen Anordnungen Besonderheiten, die weiter unten erläutert werden. Das planare Substrat 1a hat gegenüber zylindrischen Substratkonstruktionen erhebliche fertigungstechnische Vorteile; insbesondere kann es mit an sich bekannten Ätztechniken oder XYgesteuerten Mikrofräsern gemäß bekannten Techniken der Mikrostrukturtechnik in effizienter und kostengünstiger Weise erzeugt werden.

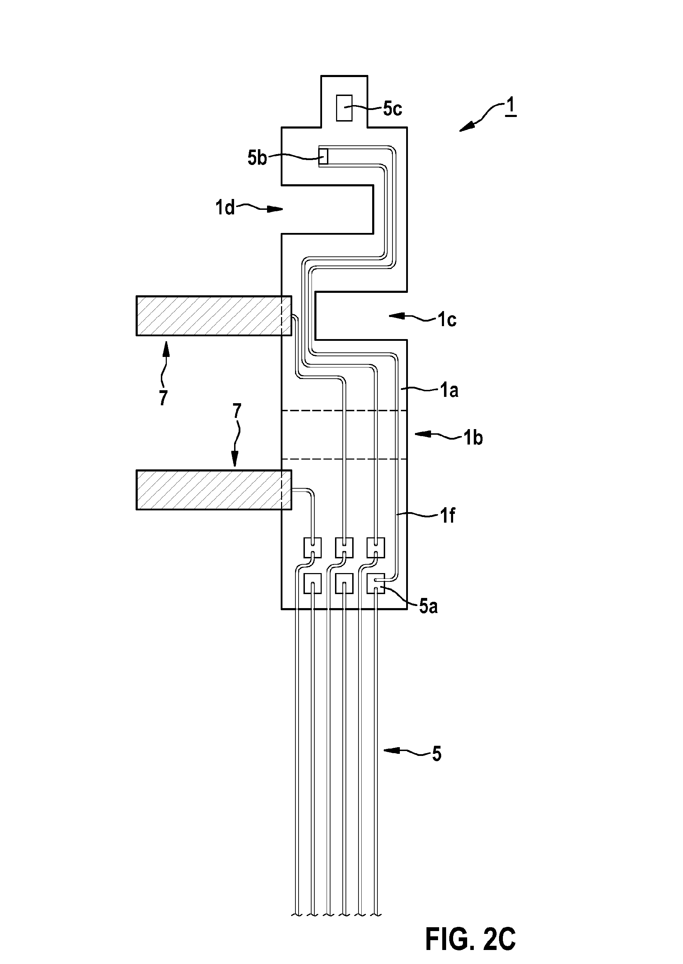

Fig. 2c zeigt beispielhaft und rein schematisch eine rückseitige Verdrahtung des Kraftum-former 1 (ohne die FBG-Faser 1e), zum Anschluss der Leiterflächen 7 sowie zweier distaler Kontaktflächen 5b, 5c, die etwa zur Stromversorgung bestimmter (nicht dargestellter) elektronischer Bauteile oder zum Anschluss einer Ablationselektrode vorgesehen sein können. Hierbei sind keine Zuleitungen zur Kontaktfläche 5c zu erkennen; solche können insbesondere in einer unter der Substratoberfläche liegenden zweiten Verdrahtungsschicht angeordnet sein. Grundsätzlich ist es aber auch möglich, auf der hier zu erkennenden Oberfläche noch weitere Leitungen vorzusehen, mit denen jene Kontaktfläche 5c mit zusätzlichen proximalen Kontaktflächen 5a verbunden würde.

Die Realisierung der Leiterbahnen zwischen den Leiterfahnen 7 und der distalen Kontaktfläche 5b und den proximalen Kontaktflächen 5a kann mit an sich bekannten Verfahren der Leiterplatten- bzw. Halbleitertechnologie erfolgen, also mit fotolithografischen Strukturierungs-, Beschichtungs- und Ätzprozessen oder in Dickschichttechnik o.ä.. Hier wirkt sich ein weiterer wesentlicher Vorteil der Erfindung aus, dass nämlich auf ausgereifte und hochproduktive planare Beschichtungs- und Strukturierungsprozesse zurückgegriffen werden kann.

Im Hinblick darauf, dass eine hochgenaue Kraftmessung die Kenntnis sensorspezifischer bzw. katheterspezifischer Daten des einzelnen Produkts in einer eingeschlossenen Auswertungseinheit erfordert, hat der Katheter gemäß einer weiteren Ausführung der Erfindung einen, insbesondere am oder nahe einem proximalen Ende angeordneten, Informationsträger mit spezifischer Kalibrierungsinformation. In einer Ausgestaltung ist der Informationsträger als RFID-Tag oder Barcode ausgebildet, welcher mit an sich bekannten und kostengünstigen Mitteln an der Signalverarbeitungseinheit leicht und zuverlässig ausgelesen werden kann.

Zur Berechnung eines 3D-Kraftvektors muss eine Kalibrierung des Sensorsystems stattfinden, indem der Sensor nacheinander mit drei zueinander senkrechten Kräften (Fx, Fy, Fz) belastet wird.

In der Theorie berechnen sich die Dehnungen $\varepsilon_1$, $\varepsilon_2$ und $\varepsilon_3$ in den verteilten Sensorbereichen mit den Biegesteifigkeiten EI, den Biegemomenten $Fl$ und den Abstand $r$ zur dehnungsneutralen Faser wie folgt (Fig. 1):

$$\varepsilon_1 \quad = \quad \frac{F_x \cdot l_1}{E \cdot l_{xx1}} \quad \cdot r_{11} \quad + \quad \frac{F_y \cdot l_1}{E \cdot l_{yy1}} \quad \cdot r_{12} \quad + \quad \frac{F_z}{A \cdot E}$$

$$\varepsilon_2 \quad = \quad \frac{F_x \cdot l_2}{E \cdot l_{xx2}} \quad \cdot r_{21} \quad + \quad \frac{F_y \cdot l_2}{E \cdot l_{yy2}} \quad \cdot r_{22} \quad + \quad \frac{F_z}{A \cdot E}$$

$$\varepsilon_3 \quad = \quad \frac{F_x \cdot l_3}{E \cdot l_{xx3}} \quad \cdot r_{31} \quad + \quad \frac{F_y \cdot l_3}{E \cdot l_{yy3}} \quad \cdot r_{32} \quad + \quad \frac{F_z}{A \cdot E}$$

Mit drei Messungen, in denen drei zueinander senkrechte Kräfte verwendet werden, kann eine entsprechende Kalibrierungsmatrix berechnet werden:

$$\begin{pmatrix} C_{11} & C_{12} & C_{13} \\ C_{21} & C_{22} & C_{23} \\ C_{31} & C_{32} & C_{33} \end{pmatrix} = \begin{pmatrix} F_x & 0 & 0 \\ 0 & F_y & 0 \\ 0 & 0 & F_z \end{pmatrix} \cdot \begin{pmatrix} \varepsilon_{11} & \varepsilon_{12} & \varepsilon_{13} \\ \varepsilon_{21} & \varepsilon_{22} & \varepsilon_{23} \\ \varepsilon_{31} & \varepsilon_{32} & \varepsilon_{33} \end{pmatrix}^{-1} \qquad \text{Gl. 2}$$

Dabei müssen die Biegesteifigkeiten $EI$ und Abstände zur neutralen Faser $r$ in dem Sensorhalter in der Art konstruiert werden, dass sich drei linear unabhängige Gleichungen ergeben. So ist es möglich einen 3D-Kraftvektor aus gemessenen Dehnungen einer beliebigen Kraftrichtung mit Hilfe der Kalibrierungsmatrix zu berechnen:

$$\begin{pmatrix} F_x \\ F_y \\ F_z \end{pmatrix} = \begin{pmatrix} C_{11} & C_{12} & C_{13} \\ C_{21} & C_{22} & C_{23} \\ C_{31} & C_{32} & C_{33} \end{pmatrix} \cdot \begin{pmatrix} \varepsilon_1 \\ \varepsilon_2 \\ \varepsilon_3 \end{pmatrix} \qquad \text{Gl. 3}$$

Diese Kalibrierungsdaten werden optional im Katheter auf einem EEPROM, RFID oder einem 2D-Barcode gespeichert und von der Auswerteeinheit drahtlos, drahtgebunden oder beim 2D-Barcode optisch ausgelesen.

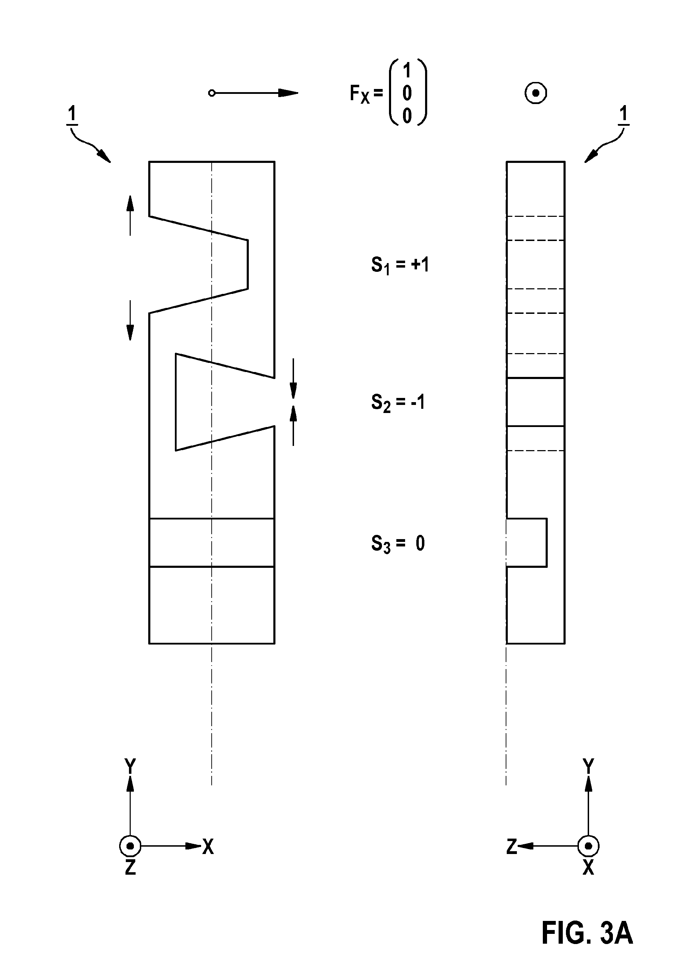

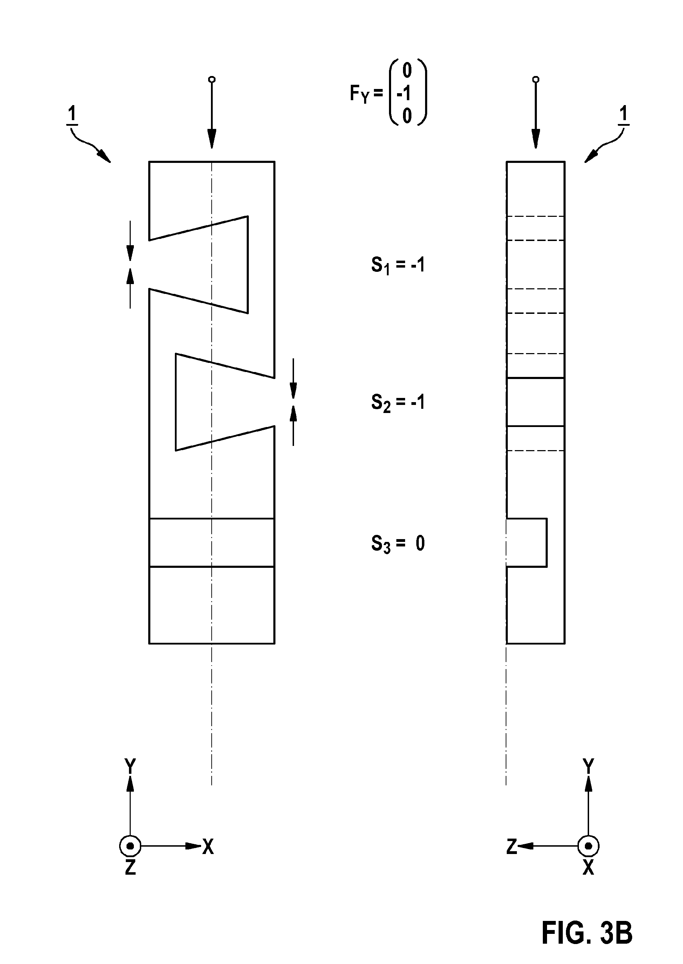

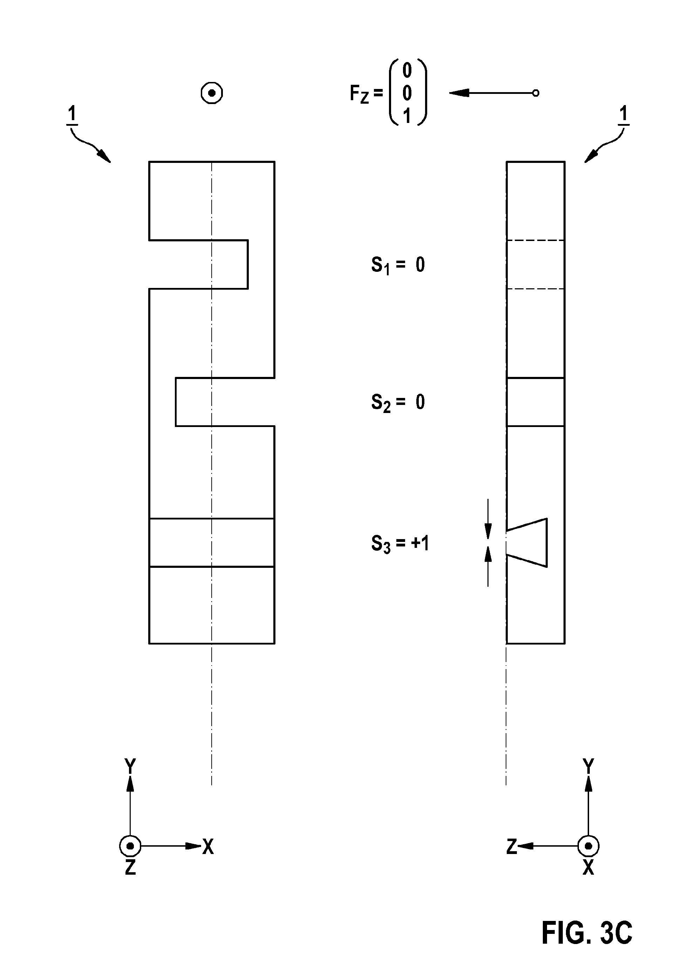

Nachfolgend und unter Bezugnahme auf Fig. 3a bis 3c werden drei Fallbeispiele zur Erläuterung der Kalibrierung eines erfindungsgemäßen Kraftumformers gegeben. Fall 1 (Fig. 3a) ist eine ausschließlich aus X-Komponente beste-hende Kraft $F_x$, Fall 2 (Fig. 3b) ist eine ausschließlich aus einer Y-Komponente bestehende Kraft Fy, und Fall 3 ist eine ausschließlich aus einer Z-Komponente bestehende Kraft $F_z$. An der jeweiligen Draufsicht bzw. Seitenansicht des Kraftumformers 1 ist mit Pfeilen gekennzeichnet, welche Verformung der einzelnen Sensorbereiche $S_1$, $S_2$ bzw. $S_3$ durch die jeweilige Kraft bewirkt wird, und es wird angenommen, dass dieser Verformung ein digitales Signal +1 bzw. -1 entspricht, während ein unverformter Sensorbereich das Signal 0 liefert.

Im Fall 1 lässt sich (im Idealfall) Gleichung 3 - unter Vertauschung der beiden Seiten - wie folgt schreiben:

$$\begin{bmatrix} +1 \\ -1 \\ 0 \end{bmatrix} * \begin{bmatrix} 0,5 & -0,5 & 0 \\ 0,5 & 0,5 & 0 \\ 0 & 0 & -1 \end{bmatrix} = \begin{bmatrix} (0,5*1) + (-0,5*-1) + 0 \\ (0,5*1) + (0,5*-1) + 0 \\ (0*1) + (0*-1) + 0 \end{bmatrix} = \begin{bmatrix} 1 \\ 0 \\ 0 \end{bmatrix} \quad \text{Gl. 3.1}$$

Im Fall 2 stellt sich die Gleichung wie folgt dar:

$$\begin{bmatrix} -1 \\ -1 \\ 0 \end{bmatrix} * \begin{bmatrix} 0,5 & -0,5 & 0 \\ 0,5 & 0,5 & 0 \\ 0 & 0 & -1 \end{bmatrix} = \begin{bmatrix} (0,5*-1) + (-0,5*-1) + 0 \\ (0,5*-1) + (0,5*-1) + 0 \\ (0*-1) + (0*-1) + 0 \end{bmatrix} = \begin{bmatrix} 0 \\ -1 \\ 0 \end{bmatrix} \quad \text{Gl. 3-2}$$

Im Fall 3 stellt sich die Gleichung wie folgt dar:

$$\begin{bmatrix} 0 \\ 0 \\ -1 \end{bmatrix} * \begin{bmatrix} 0,5 & -0,5 & 0 \\ 0,5 & 0,5 & 0 \\ 0 & 0 & -1 \end{bmatrix} = \begin{bmatrix} (0,5*0) + (-0,5*0) + (0*-1) \\ (0,5*0) + (0,5*0) + (0*-1) \\ (0*0) + (0*0) + (-1*-1) \end{bmatrix} = \begin{bmatrix} 0 \\ 0 \\ 1 \end{bmatrix} \quad \text{Gl. 3.3}$$

In der Praxis ist davon auszugehen, dass die Kraftkomponenten ungleich auf die Sensorbereiche übertragen werden; so ist etwa der Sensorbereich $S_1$ sensitiver als beispielsweise $S_2$, und der Sensorbereich $S_3$ ist auch sensibel für X- und Y-Komponenten der einwirkenden Kraft. In der Realität gibt es generell ein Übersprechen zwischen den Sensorbereichen, d.h. jeder Sensorbereich ist auch in gewissem Maße sensibel für Krafteinwirkungen, die senkrecht zur Vorzugsachse seiner Sensitivität ausgerichtet sind. Dies wird mittels einer geeigneten Koeffizientenmatrix berücksichtigt, die beispielhaft die nachfolgende Form haben kann:

$$M = \begin{bmatrix} 0,2 & -0,8 & 0 \\ 0,2 & 0,8 & 0 \\ 0,1 & 0,1 & 0,8 \end{bmatrix} \quad \text{Gl. 4}$$

Die Ausführung der Erfindung ist nicht auf die oben beschriebenen Beispiele und hervorgehobenen Aspekte beschränkt, sondern kann ebenso in einer Vielzahl von Abwandlungen erfolgen, die im Rahmen fachmännischen Handelns liegen. Beispielsweise können in der Ausführung als reines Anschlusselement - ohne Funktionalität eines Dehnmessstreifens des oben erläuterten oder eines ähnlichen Aufbaus - Ausnehmungen im Substrat etwa als Leiterzug-Unterbrechungen oder zur platzsparenden und geschützten Unterbringung von Schaltkreisen oder auch diskreten elektronischen Bauteilen dienen.

**Patentansprüche**

1.    Sensor-Anschlusselement eines Katheters oder einer Elektrodenleitung, welches ein plattenartiges Substrat (1a) mit mindestens einer Ausnehmung (1b, 1c, 1d) und schichtartigen elektrischen Leitern mit Sensor-Anschlussflächen und/oder Aktor-Anschlussflächen auf oder unterhalb mindestens einer Sensoroberfläche aufweist.

2.    Sensor-Anschlusselement nach Anspruch 1, wobei das plattenartige Substrat (1a) im Gebrauchszustand eben ist.

**3.** Sensor-Anschlusselement nach Anspruch 1, wobei das plattenartige Substrat (1a) im Gebrauchszustand insbesondere in die Form eines Hohlzylinders oder Hohlzylinderabschnitts, gebogen ist.

**4.** Sensor-Anschlusselement nach einem der vorangehenden Ansprüche, wobei das Substrat (1a) mehrschichtig aufgebaut ist und zwei oder mehr Ebenen hat, in denen schichtartige elektrische Leiter angeordnet sind.

**5.** Sensor-Anschlusselement nach einem der vorangehenden Ansprüche, wobei das Substrat (1a) aus einem drucksteifen Material gebildet ist und mindestens je eine Ausnehmung (1b, 1c, 1d) in beiden Seitenkanten und in mindestens einer Oberfläche aufweist, die dem Sensor-Anschlusselement eine vorbestimmte Biegeelastizität verleihen.

**6.** Sensor-Anschlusselement nach Anspruch 5, wobei gegeneinander in Längsrichtung des Substrats (1a) versetzte und sich bis über die Mittellinie erstreckende Ausnehmungen (1b, 1c, 1d) in den Seitenkanten vorgesehen sind, derart, dass das Sensor-Anschlusselement eine vorbestimmte Druck-Elastizität gegenüber in Längsrichtung wirkenden Kräften aufweist.

**7.** Sensor-Anschlusselement nach einem der vorangehenden Ansprüche, wobei die Ausnehmung oder mindestens eine von mehreren Ausnehmungen (1b, 1c, 1d) rechteckigen oder trapezförmigen Querschnitt hat.

**8.** Sensor-Anschlusselement nach einem der vorangehenden Ansprüche, wobei das Substrat (1a) ein Metall oder Halbleitermaterial, insbesondere Silizium, und/oder eine Keramik, insbesondere Aluminiumoxid-Keramik, und/oder ein Kunststoffinaterial, insbesondere Epoxidharz, aufweist.

**9.** Sensor-Anschlusselement nach einem der Ansprüche 6 bis 8, wobei auf dem Substrat (1a) in zu den Seitenkanten paralleler Anordnung ein FBG-Sensor (1e) angebracht und über die Sensor-Anschlussflächen derart angeschlossen ist, dass das Sensor-Anschlusselement einen 3D-Kraftsensor bildet.

**10.** Sensor-Anschlusselement nach einem der Ansprüche 6 bis 8, wobei die schichtartigen Leiter auf dem Substrat (1a) derart ausgebildet und wahlweise mit zusätzlichen Funktionselementen versehen sind, derart, dass das Sensor-Anschlusselement einen elektrischen Dehnungsmessstreifen bildet.

**11.** Sensor-Anschlusselement nach einem der vorangehenden Ansprüche, wobei auf mindestens einer Aktor-Anschlussfläche eine Leiterfahne (7) zur Realisierung einer Abfüll- und/oder Stimulationselektrode einer Elektrodenleitung aufgebracht ist.

**12.** Katheter mit einem Katheterkörper, in den ein Sensor-Anschlusselement nach einem der vorangehenden Ansprüche eingebettet ist, und einem mittels des Sensor-Anschlusselementes realisierten oder angeschlossenen Sensor und/oder Aktor.

**13.** Elektrodenleitung mit einem Leitungskörper (3b), in den ein Sensor-Anschlusselement nach einem der Ansprüche 1 bis 11 eingebettet ist, und einem mittels des Sensor-Anschlusselementes realisierten oder angeschlossenen Sensor und/oder einer mittels des Sensor-Anschlusselementes realisierten Abfühl- und/oder Stimulations- oder Ablationselektrode.

**14.** Elektrodenleitung nach Anspruch 13, mit einem Sensor-Anschlusselement nach einem der Ansprüche 9 bis 11, wobei der Leitungskörper (3b) in einem distalen Abschnitt (3a), in dem das Sensor-Anschlusselement angeordnet ist, druck- und biegeweich ausgeführt ist, derart, dass er das Ansprechen des 3D-Kraftsensors oder Dehnungsmessstreifens auf externe Kräfte nicht verfälscht.

**15.** Elektrodenleitung nach Anspruch 13 oder 14, mit mindestens einer Ringelektrode (7'), die durch Umwickeln des Leitungskörpers (3b) mit einer auf eine Aktor-Anschlussfläche des Sensor-Anschlusselementes aufgebrachte Leiterfahne (7) gebildet ist.

FIG. 1B

FIG. 1A

FIG. 2A

FIG. 2B

FIG. 2C

$$F_X = \begin{pmatrix} 1 \\ 0 \\ 0 \end{pmatrix}$$

$S_1 = +1$

$S_2 = -1$

$S_3 = 0$

**FIG. 3A**

$$F_Y = \begin{pmatrix} 0 \\ -1 \\ 0 \end{pmatrix}$$

$S_1 = -1$

$S_2 = -1$

$S_3 = 0$

1

FIG. 3B

$$F_Z = \begin{pmatrix} 0 \\ 0 \\ 1 \end{pmatrix}$$

1

$S_1 = 0$

$S_2 = 0$

$S_3 = +1$

Y
Z
X

Y
Z
X

**FIG. 3C**

**Europäisches Patentamt**
**European Patent Office**
**Office européen des brevets**

# EUROPÄISCHER RECHERCHENBERICHT

Nummer der Anmeldung

EP 15 16 8296

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2006 030407 A1 (WERTHSCHUETZKY ROLAND [DE]) 3. Januar 2008 (2008-01-03) | 1,2,7,8, 10,12,13 | INV. A61B18/14 |
| Y | * Absätze [0008], [0009], [0023], [0024], [0036] - [0038]; Abbildungen 1-12 * | 3-6,9, 11,14,15 | A61N1/06 A61N1/05 A61B5/00 G01L1/24 |
| | ----- | | A61B19/00 |
| X | US 2003/018273 A1 (CORL PAUL D [US] ET AL) 23. Januar 2003 (2003-01-23) | 1,2,4,7, 8,10,12, 13 | |
| | * Absätze [0036], [0041], [0042], [0052]; Abbildungen 1-17 * | | |
| | ----- | | |
| Y | US 5 343 860 A (METZGER WILLIAM T [US] ET AL) 6. September 1994 (1994-09-06) * Spalte 3, Zeile 18 - Zeile 30; Abbildungen 1-10 * * Spalte 5, Zeile 12 - Zeile 18 * | 3,11,15 | |
| | ----- | | |
| Y | US 2012/265102 A1 (LEO GIOVANNI [CH] ET AL) 18. Oktober 2012 (2012-10-18) * Absätze [0021], [0022], [0030], [0061], [0105] - [0107]; Abbildungen 1-14B * | 5,6 | |
| | ----- | | RECHERCHIERTE SACHGEBIETE (IPC) |
| Y | US 2008/085074 A1 (WAKAHARA MASAHITO [JP] ET AL) 10. April 2008 (2008-04-10) * Absätze [0124], [0128], [0131]; Abbildungen 1-26 * | 4,9 | A61B A61N G01L |
| | ----- | | |
| Y | DE 20 2014 100938 U1 (HECKER RAOUL [DE]) 12. März 2014 (2014-03-12) * Absatz [0083]; Abbildungen 1-11 * | 14 | |
| | ----- | | |

Der vorliegende Recherchenbericht wurde für alle Patentansprüche erstellt

| Recherchenort | Abschlußdatum der Recherche | Prüfer |
|---|---|---|
| Den Haag | 27. Oktober 2015 | Viidebaum, Mikk |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 16 8296

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-10-2015

| Im Recherchenbericht angeführtes Patentdokument | Datum der Veröffentlichung | Mitglied(er) der Patentfamilie | Datum der Veröffentlichung |
|---|---|---|---|
| DE 102006030407 A1 | 03-01-2008 | DE 102006030407 A1<br>EP    2038627 A2<br>JP    5054102 B2<br>JP 2009541752 A<br>US 2010024574 A1<br>WO 2008000246 A2 | 03-01-2008<br>25-03-2009<br>24-10-2012<br>26-11-2009<br>04-02-2010<br>03-01-2008 |
| US 2003018273 A1 | 23-01-2003 | AU    3212895 A<br>CA    2198909 A1<br>DE   69534748 T2<br>EP    0778746 A1<br>EP    1658808 A1<br>JP    3619845 B2<br>JP  H10505269 A<br>US    5715827 A<br>US    6106476 A<br>US    6767327 B1<br>US 2003018273 A1<br>US 2003040674 A1<br>US 2006094982 A1<br>US 2007135718 A1<br>US 2007149885 A1<br>US 2011251497 A1<br>WO    9607351 A1 | 27-03-1996<br>14-03-1996<br>02-11-2006<br>18-06-1997<br>24-05-2006<br>16-02-2005<br>26-05-1998<br>10-02-1998<br>22-08-2000<br>27-07-2004<br>23-01-2003<br>27-02-2003<br>04-05-2006<br>14-06-2007<br>28-06-2007<br>13-10-2011<br>14-03-1996 |
| US 5343860 A | 06-09-1994 | KEINE | |
| US 2012265102 A1 | 18-10-2012 | CN    103607961 A<br>EP    2696777 A1<br>JP 2014517740 A<br>US 2012265102 A1<br>WO 2012142588 A1 | 26-02-2014<br>19-02-2014<br>24-07-2014<br>18-10-2012<br>18-10-2012 |
| US 2008085074 A1 | 10-04-2008 | JP 2008070357 A<br>US 2008085074 A1 | 27-03-2008<br>10-04-2008 |
| DE 202014100938 U1 | 12-03-2014 | DE 102013111817 A1<br>DE 202014100938 U1<br>WO 2015059311 A1 | 30-04-2015<br>12-03-2014<br>30-04-2015 |

EPO FORM P0461

Für nähere Einzelheiten zu diesem Anhang : siehe Amtsblatt des Europäischen Patentamts, Nr.12/82

**EP 2 954 867 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080285909 A1
- EP 2491883 A1
- WO 2009007857 A2
- WO 2008503307 A2
- WO 2010088922 A1